(19) Europäisches Patentamt
European Patent Office
Office européen des brevets

(11) Veröffentlichungsnummer: **0 446 925 B1**

(12) **EUROPÄISCHE PATENTSCHRIFT**

(45) Veröffentlichungstag der Patentschrift: **21.06.95**

(51) Int. Cl.[6]: **C07C 69/92**, C07C 67/00, C07C 69/88, A61K 7/46

(21) Anmeldenummer: **91103950.1**

(22) Anmeldetag: **14.03.91**

# (54) Verfahren zur Herstellung von beta-Resorcylsäurederivaten.

(30) Priorität: **15.03.90 DE 4008223**

(43) Veröffentlichungstag der Anmeldung:
**18.09.91 Patentblatt 91/38**

(45) Bekanntmachung des Hinweises auf die Patenterteilung:
**21.06.95 Patentblatt 95/25**

(84) Benannte Vertragsstaaten:
**CH DE ES FR GB LI NL**

(56) Entgegenhaltungen:
**DE-B- 1 941 041**

**CHEMICAL ABSTRACTS, Band 77, Nr. 17, 23. Oktober 1972, S. 413, Zusammenfassung Nr. 113 983f, Columbus, Ohio, USA; T. KATO et al, "Ketene and its derivatives. XLIX. Reaction of diketene with beta-ketoesters to give ethyl orsellinate, divarate, olivetol carboxylate, and sphaeropherol carboxylate"**

(73) Patentinhaber: **Consortium für elektrochemische Industrie GmbH**
**Zielstattstrasse 20**
**D-81379 München (DE)**

(72) Erfinder: **Hirsenkorn, Rolf, Dr.**
**Pullacher Strasse 23**
**W-8023 Grosshesselohe (DE)**

EP 0 446 925 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Herstellung von β-Resorcylsäurederivaten und Verwendung bestimmter β-Resorcylsäurederivate als Riechstoffe.

Verfahren zur Herstellung von β-Resorcylsäurederivaten sind bereits bekannt. In US-PS 3,944,596 (P.F.W. Beheer B.V., Amersfoort (NL); ausgegeben am 17. März 1976) wird ein Verfahren zur Herstellung von Mono- und Dialkylresorcylsäureestern beschrieben, wobei die entsprechenden Dihydroresorcylsäureester mit N-Halogenamiden als Aromatisierungsmittel umgesetzt werden. Ein ähnliches Verfahren ist in US-PS 4,142,053 (E. Klein, Dragoco Gerberding & Co. GmbH; ausgegeben am 27. Februar 1979) offenbart, wonach die Dihydro-β-Resorcylsäureester durch Erhitzen mit Acetanhydrid/Schwefelsäure unter Bildung von Diacetaten aromatisiert werden und diese Diacetate alkalisch oder sauer in einer zusätzlichen Reaktionsstufe verseift werden. Diese Verfahren haben den Nachteil, daß die Ausgangsstoffe relativ schwer zugänglich sind und in der Regel zusätzliche Syntheseschritte benötigt werden.

Des weiteren können β-Resorcylsäureester gemäß US-PS 4,420,629 (H. Schmidt, Dynamit Nobel AG; ausgegeben am 13. Dezember 1983) durch Umsetzung von α-Pyronyl-6-essigsäureester mit einer Base hergestellt werden, wobei die α-Pyronyl-essigsäureester gemäß DE-PS 29 16 648 (H. Schmidt; Dynamit Nobel AG; ausgegeben am 6. November 1980) hergestellt werden. Dieses Verfahren hat den Nachteil, daß es ebenfalls mehrstufig ist und von nicht leicht zugänglichen Edukten ausgeht.

Ferner ist aus einer Veröffentlichung von Tetsuzo Kato und Toyaharu Hozumi in Chem. Pharm. Bull. 20, 7 (1972) 1574-8 ein Verfahren zur Herstellung von 6-Alkyl-β-Resorcylsäureester durch Umsetzung von Diketen (4-Methylen-2-oxetanon) mit β-Ketocarbonsäureester und Natriumhydrid im Molverhältnis 1:1:1 bekannt. Dieses Verfahren führt lediglich zu niedrigen Ausbeuten und hat noch den Nachteil, daß das als Katalysator verwendete Natriumhydrid schwer handhabbar ist.

Es bestand daher die Aufgabe, ein Verfahren bereitzustellen, welches es erlaubt, β-Resorcylsäurederivate auf relativ einfache Weise und mit einer hohen Ausbeute herzustellen. Diese Aufgabe wird durch die Erfindung gelöst.

Gegenstand der Erfindung ist ein Verfahren zur Herstellung von β-Resorcylsäurederivaten der allgemeinen Formel

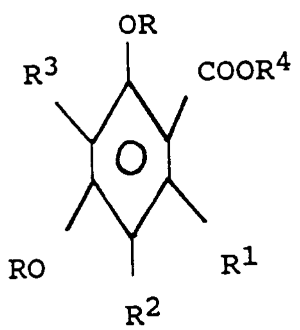

(I),

worin R, $R^2$ und $R^3$ gleich oder verschieden sein können und Wasserstoffatom oder einen Alkylrest bedeuten und $R^1$ und $R^4$ gleich oder verschieden sein können und einen Alkylrest bedeuten,
durch Umsetzung von Diketen der allgemeinen Formel

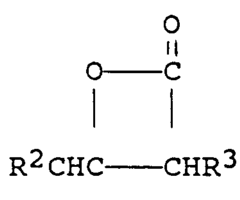

(II),

in welcher $R^2$ und $R^3$ die oben angegebene Bedeutung haben, mit β-Ketocarbonsäurederivaten der allgemeinen Formel

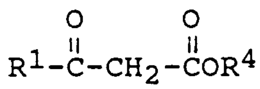 (III),

in welcher $R^1$ und $R^4$ die oben angegebene Bedeutung haben, in Anwesenheit von Erdalkalimetallverbindung.

Bei dem erfindungsgemäßen Verfahren werden vorzugsweise solche Diketene der allgemeinen Formel (II) eingesetzt, bei denen $R^2$ und $R^3$ gleich oder verschieden sein können und Wasserstoffatom oder Alkylgruppe mit 1 bis 5 Kohlenstoffatomen bedeuten.

Beispiele für Kohlenwasserstoffreste mit 1 bis 5 Kohlenstoffatomen sind Methyl-, Ethyl-, n-Propyl- iso-Propyl-, 1-n-Butyl-, iso-Butyl-, 2-n-Butyl-, tert.-Butyl-, n-Pentyl-, iso-Pentyl-, tert.-Pentyl- und neo-Pentylreste.

Besonders bevorzugt werden bei dem erfindungsgemäßen Verfahren Diketene der allgemeinen Formel (II) eingesetzt, bei denen $R^2$ und $R^3$ gleich oder verschieden sein können und Wasserstoffatom oder Alkylgruppe mit 1 bis 3 Kohlenstoffatomen, insbesondere Wasserstoffatom und Methylrest, bedeuten.

Beispiele für Reste R sind die für die Reste $R^2$ bzw. $R^3$ genannten Reste.

Bevorzugte Reste R sind Wasserstoffatom, Methyl- und Ethylrest, wobei Wasserstoffatom und der Methylrest besonders bevorzugt sind.

Beispiele für die im erfindungsgemäßen Verfahren eingesetzten Diketene gemäß Formel (II) sind

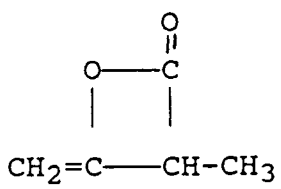 , 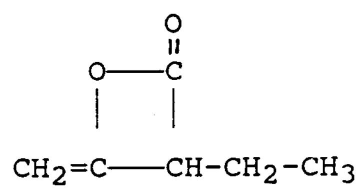 ,

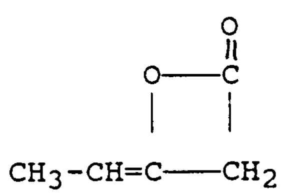 , 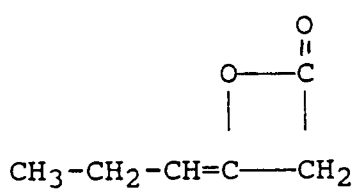 ,

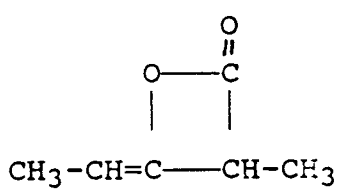 , 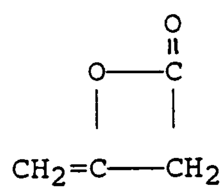 und

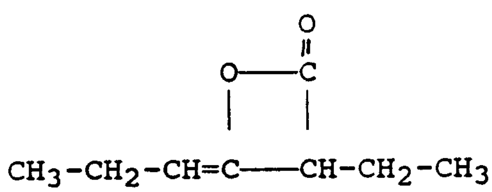 .

Beispiele für die im erfindungsgemäßen Verfahren besonders bevorzugt eingesetzten Diketene sind

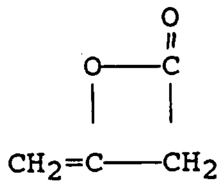 , 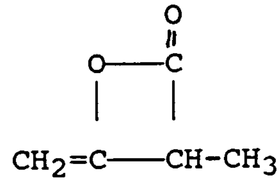

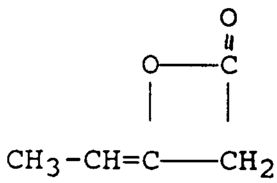 und 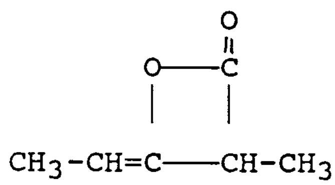 .

Bei den erfindungsgemäß eingesetzten Diketenen kann es sich um eine einzelne Art eines Diketens handeln. Es kann sich aber auch um ein Gemisch aus mindestens zwei Arten derartiger Diketene handeln.

Die erfindungsgemäß eingesetzten Diketene können nach an sich bekannten Verfahren hergestellt werden. Hierzu sei beispielsweise auf US-PS 2,238,826 (E.I. du Pont de Nemours & Co.; ausgegeben am 15. April 1941) und J.D. Roberts, J.Am. Chem. Soc. 71 (1949) 843-7 verwiesen.

Vorzugsweise werden die im erfindungsgemäßen Verfahren eingesetzten Diketene dadurch hergestellt, daß Carbonsäurehalogenid der allgemeinen Formel

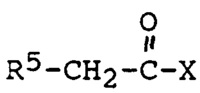 (IV),

worin X Halogenatom, wie Fluor, Chlor, Brom oder Jod, besonders bevorzugt Chlor, bedeutet und $R^5$ eine der Bedeutungen von $R^2$ bzw. $R^3$ hat,
in Anwesenheit von Base, wie beispielsweise Triethylamin, Pyridin, N-Methylmorpholin und Diisopropylethylamin, besonders bevorzugt Triethylamin, umgesetzt wird. Bei dem eingesetzten Carbonsäurehalogenid kann es sich dabei um eine einzelne Art eines Carbonsäurehalogenids wie auch um ein Gemisch aus mindestens zwei Arten derartiger Carbonsäurehalogenide handeln.

Obwohl in Formel (III) nicht angegeben, können als β-Ketocarbonsäurederivate auch Derivate, wie beispielsweise solche der allgemeinen Formeln

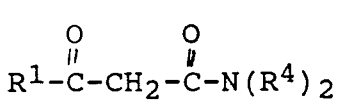 oder 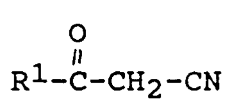

mit $R^1$ und $R^4$ gleich eine der obengenannten Bedeutungen eingesetzt werden, wobei dann das nach der erfindungsgemäßen Umsetzung erhaltene Produkt anschließend noch in ein β-Resorcylsäurederivat gemäß der allgemeinen Formel (I) umgearbeitet werden muß.

Bei dem erfindungsgemäßen Verfahren werden vorzugsweise solche β-Ketocarbonsäurederivate der allgemeinen Formel (III) eingesetzt, bei denen $R^1$ und $R^4$ gleich oder verschieden sein können und eine Alkylgruppe mit 1 bis 5 Kohlenstoffatomen bedeuten.

Beispiele für die Reste $R^1$ bzw. $R^4$ sind alle die für die Reste $R^2$ bzw. $R^3$ angegebenen Beispiele.

Besonders bevorzugt werden bei dem erfindungsgemäßen Verfahren β-Ketocarbonsäurederivate der allgemeinen Formel (III), bei denen $R^1$ und $R^4$ gleich oder verschieden sein können und eine Alkylgruppe mit 1 bis 3 Kohlenstoffatomen, insbesondere den Methylrest, bedeuten, eingesetzt.

Beispiele für die im erfindungsgemäßen Verfahren eingesetzten β-Ketocarbonsäurederivate gemäß Formel (III) sind

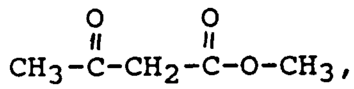

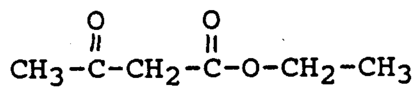 und

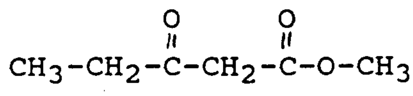 .

Im erfindungsgemäßen Verfahren wird als β-Ketocarbonsäurederivat besonders bevorzugt

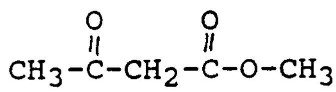

eingesetzt.

Bei den erfindungsgemäß eingesetzten β-Ketocarbonsäurederivaten kann es sich um eine einzelne Art eines β-Ketocarbonsäurederivats handeln. Es kann sich aber auch um ein Gemisch aus mindestens zwei Arten derartiger β-Ketocarbonsäurederivate handeln.

Die im erfindungsgemäßen Verfahren eingesetzten β-Ketocarbonsäurederivate sind handelsübliche Produkte bzw. lassen sich nach in der organischen Chemie gängigen Methoden herstellen. Hierzu sei beispielsweise auf G. Hesse, in Houben-Weyl, Methoden der organischen Chemie, "Enole"; Thieme Verlag, Stuttgart, 1968, 4. Auflage, Bd. VI/1d, Seite 73 ff und E.J. Roskamp, J. Org. Chem. 54 (1989) 3258-60 verwiesen.

Bei der im erfindungsgemäßen Verfahren eingesetzten Erdalkalimetallverbindung handelt es sich vorzugsweise um organische und anorganische Verbindungen des Magnesiums, Calciums, Strontiums und Bariums.

Beispiele für die im erfindungsgemäßen Verfahren eingesetzten Erdalkalimetallverbindungen sind Erdalkalihydroxide, wie $Mg(OH)_2$, $Ca(OH)_2$, $Sr(OH)_2 \cdot xH_2O$ und $Ba(OH)_2 \cdot xH_2O$, Erdalkalioxide, wie $MgO \cdot xH_2O$ und CaO, Erdalkalicarbonate, wie $MgCO_3$, $MgHCO_3$, $CaCO_3$, $SrCO_3$ und $BaCO_3$, sowie um Erdalkalimetallalkoholate, wie beispielsweise $Mg(OC_2H_5)_2$.

Besonders bevorzugt handelt es sich bei der erfindungsgemäß eingesetzten Erdalkalimetallverbindung um Erdalkalimetalloxide und Erdalkalimetallhydroxide, insbesondere um Calciumoxid und Calciumhydroxid.

Bei der erfindungsgemäß eingesetzten Erdalkalimetallverbindung kann es sich um eine einzelne Art einer Erdalkalimetallverbindung handeln. Es kann sich aber auch um ein Gemisch aus mindestens zwei Arten derartiger Erdalkalimetallverbindungen handeln.

Bei dem erfindungsgemäßen Verfahren liegt das Molverhältnis von Diketen gemäß Formel (II) zu β-Ketocarbonsäurederivat gemäß Formel (III) zu Erdalkalimetallverbindung in der Reaktionsmasse vorzugsweise bei 1:1:1. Das Molverhältnis von Diketen gemäß Formel (II) zu β-Ketocarbonsäurederivat gemäß Formel (III) zu Erdalkalimetallverbindung kann jedoch auch geringfügig vom stöchiometrischen Verhältnis 1:1:1 abweichen.

Das erfindungsgemäße Verfahren kann in Anwesenheit oder in Abwesenheit von Lösungsmitteln durchgeführt werden, wobei die Verwendung von in bezug auf die Reaktionsmasse inertem organischen Lösungsmittel bevorzugt wird.

Beispiele für Lösungsmittel sind Alkohole, wie Methanol, Ethanol, n-Propanol, iso-Propanol, n-, sec.- und tert.-Butanol, 2-Butanol und Ethylenglycol; Ester, wie Methylacetat, Ethylacetat, n- und iso-Propylacetat, n-, sec.- und tert.-Butylacetat und Ethylformiat; Ether, wie Dioxan, Tetrahydrofuran, Diethylether, Di-n-propylether, Diisopropylether, Di-n-butylether, Ethylenglycoldiethylether, Ethylenglycoldimethylether, Diethylenglycoldimethylether, Diethylenglycoldiethylether, Diethylenglycoldibutylether und Anisol; chlorierte Kohlenwasserstoffe, wie Dichlormethan, Trichlormethan, Tetrachlormethan, 1,2-Dichlorethan, 1,1,1-Trichlorethan, Trichlorethylen, Tetrachlorethylen und Chlorbenzol; Kohlenwasserstoffe, wie Pentan, n-Hexan, Hexan-Isomerengemische, Cyclohexan, Heptan, Octan, Waschbenzin, Petrolether, Benzol, Ethylbenzol, Toluol, Xylole; Ketone, wie Aceton, Methylethylketon und Methylisobutylketon; Dimethylformamid, Dimethylsulfoxid und Gemische dieser Lösungsmittel, wobei Ether und etherhaltige Lösungsmittelgemische, insbesonders Tetrahydrofuran und Ethylenglycoldimethylether, besonders bevorzugt sind.

Die Bezeichnung Lösungsmittel bedeutet nicht, daß sich alle Reaktionskomponenten in diesen lösen müssen. Die Reaktion kann auch in einer Suspension oder Emulsion eines oder mehrerer Reaktionspartner durchgeführt werden.

Bei dem erfindungsgemäßen Verfahren wird Lösungsmittel vorzugsweise in Mengen 40 bis 90 Gewichtsprozent, besonders bevorzugt 60 bis 70 Gewichtsprozent, jeweils bezogen auf das Gesamtgewicht der Reaktionsmasse, eingesetzt.

Das erfindungsgemäße Verfahren wird vorzugsweise bei einer Temperatur zwischen 0 und 200 °C, besonders bevorzugt bei der Siedetemperatur des verwendeten Lösungsmittels, und einem Druck zwischen 900 und 1100 hPa durchgeführt. Falls erwünscht, können auch höhere oder niedrigere Drücke angewendet werden.

Bei dem erfindungsgemäßen Verfahren werden Diketen gemäß Formel (II), β-Ketocarbonsäurederivat gemäß Formel (III), Erdalkalimetallverbindung und gegebenenfalls Lösungsmittel auf beliebige Weise vermischt.

In der Regel fällt bei dem erfindungsgemäßen Verfahren β-Resorcylsäurederivat gemäß Formel (I) mit R gleich Erdalkalimetallion an, aus welchem durch Ansäuern mit wäßriger Mineralsäure, wie beispielsweise Salzsäure, Schwefelsäure und Phosphorsäure, oder organischer Säure, wie beispielsweise Essigsäure, β-Resorcylsäureester gemäß Formel (I) mit R gleich Wasserstoffatom erhalten wird.

Falls das erfindungsgemäße Verfahren in Alkanol als Lösungsmittel durchgeführt wird, können β-Resorcylderivate entstehen, deren Hydroxylgruppen teilweise oder vollständig verethert sind.

Bei einer bevorzugten Ausführungsform des erfindungsgemäßen Verfahrens wird β-Ketocarbonsäurederivat gemäß Formel (III) mit der äquimolaren Menge an Erdalkalimetallverbindung und Lösungsmittel vermischt, auf die Siedetemperatur des Lösungsmittels erhitzt und mit der äquimolaren Menge, bezogen auf die Menge an eingesetztem β-Ketocarbonsäurederivat, an Diketen versetzt.

Bei einer besonders bevorzugten Ausführungsform des erfindungsgemäßen Verfahrens wird Acetessigsäurederivat mit der äquimolaren Menge an Erdalkalimetalloxid oder Erdalkalimetallhydroxid und Tetrahydrofuran und/oder Ethylenglycoldimethylether enthaltendem Lösungsmittel vermischt, auf die Siedetemperatur des Tetrahydrofuran und/oder Ethylenglycoldimethylether enthaltenden Lösungsmittels erhitzt und mit einer äquimolaren Menge, bezogen auf die Menge an eingesetztem Acetessigsäurederivat, eines Gemisches von Diketen enthaltend 4-Methylen-2-oxetan und 3-Methyl-4-methylen-2-oxetan versetzt, wobei nach der Umsetzung organische oder anorganische Säure zugegeben wird.

Das nach dem erfindungsgemäßen Verahren hergestellte β-Resorcylsäurederivat gemäß Formel (I) kann nach an sich bekannten Verfahren, wie Extraktion, Kristallisation, Destillation und Extraktion, isoliert bzw. gereinigt werden.

Das erfindungsgemäße Verfahren hat den Vorteil, daß β-Resorcylsäurederivate der allgemeinen Formel (I) auf einfache Weise und in einem Syntheseschritt hergestellt werden können, wobei von relativ leicht zugänglichen Ausgangsstoffen ausgegangen werden kann.

Des weitern hat das erfindungsgemäße Verfahren den Vorteil, daß β-Resorcylsäurederivate der allgemeinen Formel (I) in hoher Ausbeute und mit sehr guten olfaktorischen Eigenschaften erhalten werden.

Die erfindungsgemäß hergestellten β-Resorcylsäurederivate können überall dort eingesetzt werden, wo auch bisher β-Resorcylderivate eingesetzt worden sind. So können sie beispielsweise direkt oder als Intermediate in den Bereichen Pharmazeutika, Agrochemikalien, Riechstoffe und Polymere eingesetzt werden.

Insbesondere eignet sich 3,6-Dimethyl-β-resorcylsäuremethylester aufgrund ihres charakteristischen Eichenmoosgeruchs als Duftstoffe. Eichenmoosriechstoffe sind wichtige Bestandteile in Parfums mit Chypre- und Fougère-Noten. Es wurde gefunden, daß ein Gemisch aus 3,6-Dimethyl-β-resorcylsäuremethylester und 6-Methyl-β-resorcylsäuremethylester, welches sich durch einen moosig, holzig, phenolischen Geruch auszeichnet, ein sehr guter Riechstoff mit Eichenmooscharakter ist.

6-Methyl-β-resorcylsäuremethylester eignet sich auch zur Herstellung von Orcinyl, das als Riechstoff mit Eichenmooscharakter eingesetzt werden kann. Hierzu sei beispielsweise auf G. Nicollier, M. Rebetez, R. Tabacchi, Helv.Chim.Acta 61 Fasc. 8 (1978)-Nr. 275, 2899-904 verwiesen. Des weiteren kann 6-Methyl-β-resorcylsäuremethylester auch zur Herstellung von 4-Acetoxy-6-methyl-acetylsalicylsäuremethylester, der ebenfalls als Riechstoff mit Eichenmooscharakter bekannt ist, eingesetzt werden. Hierzu sei beispielsweise

auf US-PS 3,884,843 (Fritzsche Dodge & Olcott Inc., ausgegeben am 20. Mai 1975) verwiesen.

In den nachstehend beschriebenen Beispielen beziehen sich alle Angaben von Teilen und Prozentsätzen, falls nicht anders angegeben, auf das Gewicht. Sofern nicht anders angegeben, werden die nachstehenden Beispiele bei einem Druck der umgebenden Atmosphäre, also etwa bei 1000 hPa, und bei Raumtemperatur, also bei etwa 23 °C, bzw. bei einer Temperatur, die sich beim Zusammengeben der Reaktanden bei Raumtemperatur ohne zusätzliche Heizung oder Kühlung einstellt, durchgeführt.

**Beispiel 1**

Zu einer Suspension von 27,3 g (0,48 Mol) Calciumoxid in 350 ml Tetrahydrofuran werden 55,7 g (0,48 mol) Acetessigsäuremethylester gegeben, auf 50 °C erhitzt und 1 Stunde gerührt. Anschließend werden 40,3 g (0,48 mol) 4-Methylen-2-oxetan (herstellbar nach DE-PS 687 065 (Consortium für elektrochemische Industrie GmbH, ausgegeben am 22. Januar 1940)) langsam zugetropft, wobei während des Zutropfens so gekühlt wird, daß die Temperatur des Reaktionsgemisches einen Wert von 40 °C nicht übersteigt und zwischen 30 und 40 °C liegt, 8 Stunden unter Rückfluß gekocht und nach Abkühlen auf Raumtemperatur das Tetrahydrofuran durch Destillation entfernt. Danach werden 260 ml Methyl-tert.-butylether hinzugegeben und die so erhaltene Mischung mit so viel 2N HCl versetzt bis sich das im Gemisch enthaltene Calciumoxid auflöst. Nach dreimaliger Extraktion mit Methyl-tert.-butylether wird zur organischen Phase Wasser gegeben, die wäßrige Phase mit NaOH (10 %ig) auf pH 6 eingestellt und dreimal mit Methyl-tert.-butylether extrahiert. Aus der organischen Phase erhält man nach Entfernen des Lösungsmittels 77,3 g hellbrauner Kristalle mit einem Reinheitsgrad von 92% (Gaschromatographie), was einer Ausbeute von 83% entspricht, der folgenden Struktur

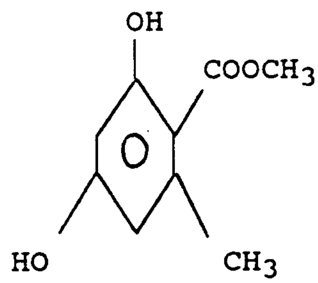

Der Geruch des so erhaltenen 6-Methyl-β-Resorcylsäuremethylesters kann wie folgt beschrieben werden: moosig, holzig, phenolisch, Eichenmooscharakter.

Der so erhaltene 6-Methyl-β-resorcylsäureester zeigt im 200 MHz-$^1$H-NMR-Spektrum in $CDCl_3$ und TMS als innerem Standard Signale bei 11,75 (s, 1H, OH), 6,27 und 6,23 (d, 1H, $J_{3/5}$ = 2Hz), 5,40 (sb, 1H, OH), 3,92 (s, 3H, $COOCH_3$) und 2,48 (s, 3H, $CH_3$).

Zur weiteren Reinigung des so erhaltenen 6-Methyl-β-resorcylsäureesters kann aus Petroleumbenzin/Ethylacetat umkristallisiert werden. Der so erhaltene 6-Methyl-β-resorcylsäureester kann beispielsweise folgendermaßen weiterverwendet werden:

a) Herstellung von Orcinyl

18,55 g des erfindungsgemäß hergestellten 6-Methyl-β-resorcylsäureesters und 14,3 g Kaliumcarbonat (wasserfrei) werden in Aceton vorgelegt, 9,5 ml Methyliodid in Aceton zugetropft und 12 Stunden unter Rückfluß gekocht. Anschließend wird das Aceton abdestilliert, unter Eiskühlung angesäuert und dreimal mit Ethylacetat extrahiert. Es werden 15,71 g (Ausbeute: 79%) 4-Methoxy-6-methyl-salicylsäuremethylester erhalten, dessen Geruch wie folgt charakterisiert werden kann: süß, erdig, Walnuß, moosig.

1,0 g des so erhaltenen 4-Methoxy-6-methyl-salicylsäuremethylesters werden mit 35 ml einer wäßrigen Kaliumhydroxydlösung (15 %ig) 8 Stunden unter Rückfluß gekocht. Anschließend wird das Reaktionsgemisch mit verdünnter Salzsäure angesäuert und mit Ethylacetat extrahiert. Es werden 0,70 g (Ausbeute: 70 %) Orcinyl (3-Methoxy-5-methylphenol) erhalten.

b) Herstellung von 4-Acetoxy-6-methyl-acetylsalicylsäuremethylester

5 g des erfindungsgemäß hergestellten 6-Methyl-β-resorcylsäureesters, 11,2 g Essigsäureanhydrid, 0,3 g Natriumacetat und 50 ml Xylol werden mehrere Stunden auf 135 °C erhitzt, wobei circa 15 ml eines Azeotrops überdestilliert. Anschließend werden das überschüssige Essigsäureanhydrid und Xylol bei 20 mmHg abdestilliert, der Rückstand in 50 ml Toluol gelöst, die organische Phase zweimal mit einer gesättigten Natriumcarbonatlösung und zweimal mit Wasser gewaschen. Der nach Entfernen des Lösungsmittels erhaltene Kristallbrei wird aus Xylol/Propanol umkristallisiert. Es werden 5,2 g 4-Acetoxy-6-methyl-acetylsalicylsäuremethylester (Ausbeute: 71 %; Schmelzpunkt: 48 °C) erhalten.

**Beispiel 2**

a) Herstellung von Diketenen

647,7 g (7,0 mol) Propionylchlorid und 549,5 g (7,0 mol) Acetylchlorid werden einem Gemisch aus 1416,7 g (14 mol) Triethylamin in 15 l Methyl-tert.-butylether zugetropft, wobei sich das Gemisch erwärmt. Nach 5-stündigem Rühren bei Raumtemperatur wird das so erhaltene Gemisch auf eine Temperatur von -15 °C abgekühlt und anschließend das Triethylaminhydrochlorid durch Absaugen abgetrennt. Die verbleibende Lösung wird auf ein Volumen von ca. 1 l eingeengt und anschließend über eine 30 cm Vigreux-Kolonne feindestilliert. Es werden 609,0 g eines Gemisches mit folgender Zusammensetzung erhalten, was einer Ausbeute von 89% entspricht:

4-Methylen-2-oxetan (DIKE) 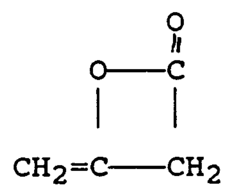 (21,4 %)

3-Methyl-4-methylen-2-oxetan (MEDIKE) 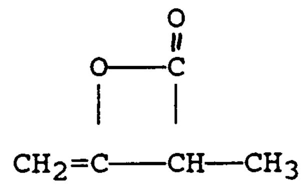 (29,7 %)

4-Ethyliden-2-oxetan (I-MEDIKE) 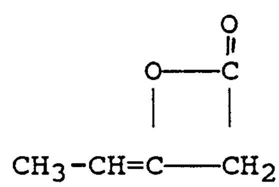 (22,6 %)

3-Methyl-4-ethyliden-2-oxetan (DIMEDIKE) 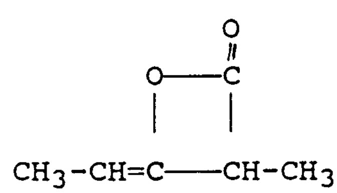 (26,2 %)

Die so erhaltenen Diketene zeigen im 200 MHz-$^1$H-NMR-Spektrum in $CDCl_3$ und TMS als internem Standard Signale bei:

DIKE: 4,80 (m, 1$H^{vin}$), 4,48 (m, 1H, $H^{vin}$), 3,88 (m, 2H, $CH_2CO$) ppm,

MEDIKE: 4,74 (dd, 1H, $H^{vin}$, $J_{gem.}$ = 5 Hz, $J_{all.}$ = 1 Hz), 4,47 (dd, 1H, $H^{vin}$, $J_{gem.}$ = 5 Hz, $J_{all.}$ = 1 Hz), 4,0 (q, 1H, $CHCH_3CO$, J = 8 Hz), 1,47 (d, 3H, $CH_3$, J = 8 Hz) ppm,

DIMEDIKE: 4,75 (dq, 1H, $H^{vin}$, $J_{vic}$ = 7 Hz, $J_{all.}$ = 1 Hz), 3,98 (dq, 1H, $CHCH_3CO$, $J_{vic}$ = 9,0 Hz, $J_{all.}$ = 1 Hz), 1,67 (dd, 3H, $CH_3$-C = C, $J_{vic}$ = 7 Hz, $J_{homoall.}$ = 1 Hz), 1,41 (d, 3H, CH $CH_3CO$, $J_{vic}$ = 9,0 Hz) ppm und

I-MEDIKE: 4,75 (q, 1H, $H_{vin}$), 4,83 (m, 2H, $CH_2$ CO), 1,7 (d, 3H, $CH_3C=C$, J = 7,0 Hz) ppm.

Das so erhaltene Diketen-Gemisch wird destillativ getrennt. Es werden die folgenden Fraktionen erhalten:

| Fraktion | | Siedebereich (20 mm Hg) |
|---|---|---|
| 1 | DIKE/MEDIKE | 40-44°C |
| 2 | I-MEDIKE/DIMEDIKE | 46-51°C |

Zu einer Suspension von 89,6 g (1,60 mol) Calciumoxid in 2,0 l Tetrahydrofuran werden 185,6 g (1,60 mol) Acetessigsäuremethylester gegeben, auf 50 °C erhitzt und 1 Stunde gerührt. Anschließend werden 145,7 g (1,60 mol) eines Gemisches aus 48 Prozent DIKE und 52 Prozent MEDIKE, deren Herstellung oben unter a) beschrieben ist, langsam zugetropft und wie in Beispiel 1 beschrieben verfahren. Aus der organischen Phase wird das Lösungsmittel entfernt und aus 1 l Toluol fraktioniert kristallisiert. Es verbleiben 266,5 g farbloser Kristalle mit einem Reinheitsgrad von 95% (Gaschromatographie), was einer Ausbeute von 88% entspricht, der folgenden Struktur

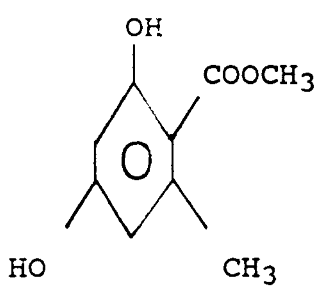

46%

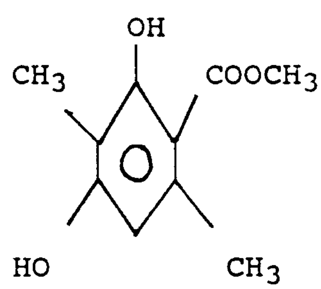

54%

Der Geruch des so erhaltenen Gemisches aus 6-Methyl-β-Resorcylsäuremethylester und 3,6- Dimethyl-β-Resorcylsäuremethylester kann wie folgt beschrieben werden: moosig, holzig, phenolisch. Das Gemisch eignet sich sehr gut als Riechstoff mit Eichenmooscharakter.

Das so erhaltene β-Resorcylsäure-methylestergemisch zeigt im 200 MHz-$^1$H-NMR-Spektrum in $CDCl_3$ und TMS als innerem Standard Signale für 6-Methyl-β-resorcylsäuremethylester bei 11,75 (s, 1H, OH), 6,27 und 6,23 (d, 1H, $J_{3/5}$ = 2Hz), 5,40 (sb, 1H, OH), 3,92 (s, 3H, $COOCH_3$) und 2,48 (s, 3H, $CH_3$) ppm und für 3,6-Dimethyl-β-resorcylsäuremethylester bei 12,05 (s, 1H, 2-OH), 6,22 (s, 1H, $H^5$), 5,19 (s, 1H, 4-OH), 3,92 (s, 3H, $COOCH_3$), 2,46 (s, 3H, 6-$CH_3$), 2,10 (s, 3H, 3-$CH_3$) ppm.

Zur weiteren Reinigung des so erhaltenen Gemisches kann noch aus Toluol, Dichlormethan oder Hexan umkristallisiert werden.

**Beispiel 3**

Zu einer Suspension von 89,6 g (1,60 mol) Calciumoxid in 2,0 l Tetrahydrofuran werden 185,6 g (1,60 mol) Acetessigsäuremethylester gegeben, auf 50 °C erhitzt und 1 Stunde gerührt. Anschließend werden

1,60 mol eines Gemisches aus 49 Prozent I-MEDIKE und 51 Prozent DIMEDIKE, deren Herstellung in Beispiel 2 unter a) beschrieben ist, langsam zugetropft und wie in Beispiel 1 beschrieben verfahren. Aus der organischen Phase wird das Lösungsmittel entfernt und aus 1 1 Toluol fraktioniert kristallisiert. Es werden farblose Kristalle mit einer Ausbeute von 91 Prozent der folgenden Struktur

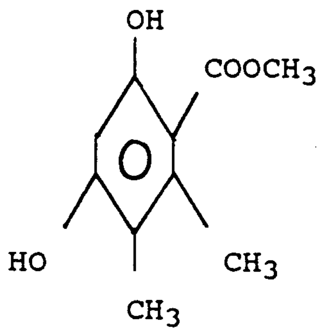

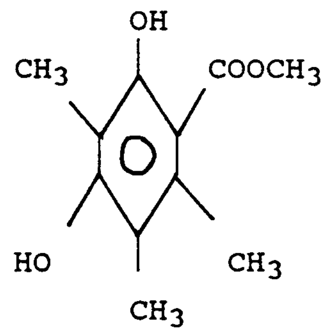

erhalten.

Bei dem so erhaltenen Gemisch aus 5,6-Dimethyl-$\beta$-Resorcylsäuremethylester (balsamisch, ambriert, fruchtig) und 3,5,6-Trimethyl-$\beta$-Resorcylsäuremethylester (Liebstöckl, Walnuß, maple lacton) dominiert der Geruch nach Liebstöckl.

Das so erhaltene $\beta$-Resorcylsäure-methylestergemisch zeigt im 200 MHz-$^1H$-NMR-Spektrum in $CDCl_3$ und TMS als innerem Standard Signale für 5,6-Dimethyl-$\beta$-resorcylsäuremethylester bei 11,26 (s,1H,2-OH), 6,27 (s, 1H, $H^3$), 5,60 (sb, 1H, OH), 3,93 (s, 3H, $COOCH_3$), 2,45 (s, 3H, 6-$CH_3$), 2,11 (s, 3H, 5-$CH_3$) ppm und für 3,5,6-Trimethyl-$\beta$-Resorcylsäuremethylester bei 11,46 (s, 1H, 2-OH) 5,15 (s, 1H, 4-OH), 3,91 (s, 3H, $COOCH_3$), 2,42 (s, 3H, 6-$CH_3$), 2,13 (s, 6H, 3-und 5-$CH_3$) ppm. $R_f$-Werte (Petroleumbenzin/Ethylacetat 8:2) für 5,6-Dimethyl-$\beta$-resorcylsäuremethylester: 0,29 und für 3,5,6-Trimethyl-$\beta$-Resorcylsäuremethylester: 0,40.

Zur weiteren Reinigung des so erhaltenen Gemisches kann noch aus Toluol, Dichlormethan oder Hexan umkristallisiert werden.

## Patentansprüche

**1.** Verfahren zur Herstellung von $\beta$-Resorcylsäurederivaten der allgemeinen Formel

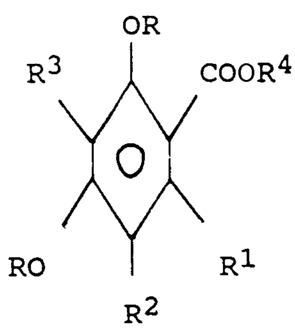

(I),

worin R, $R^2$ und $R^3$ gleich oder verschieden sein können und Wasserstoffatom oder einen Alkylrest bedeuten und $R^1$ und $R^4$ gleich oder verschieden sein können und einen Alkylrest bedeuten,
durch Umsetzung von Diketen der allgemeinen Formel

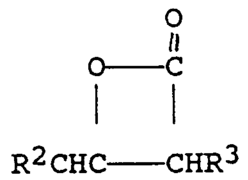

(II),

in welcher $R^2$ und $R^3$ die oben angegebene Bedeutung haben, mit β-Ketocarbonsäurederivaten der allgemeinen Formel

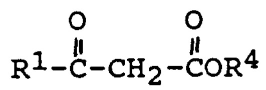

(III),

in welcher $R^1$ und $R^4$ die oben angegebene Bedeutung haben,
in Anwesenheit von Erdalkalimetallverbindung.

2. Verfahren nach Anspruch 1, dadurch gekennzeichnet, daß Diketene der allgemeinen Formel (II) eingesetzt werden, bei denen $R^2$ und $R^3$ gleich oder verschieden sein können und Wasserstoffatom oder Alkylgruppe mit 1 bis 3 Kohlenstoffatomen bedeuten.

3. Verfahren nach Anspruch 1 oder 2, dadurch gekennzeichnet, daß Diketene der allgemeinen Formel (II) durch Umsetzung von carbonsäurehalogenid der allgemeinen Formel

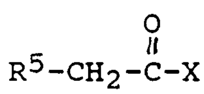

(IV),

worin X Halogenatom bedeutet und $R^5$ eine der Bedeutungen von $R^2$ bzw. $R^3$ hat, in Anwesenheit von Base hergestellt werden.

4. Verfahren nach einem oder mehreren der Ansprüche 1 bis 3, dadurch gekennzeichnet, daß β-Ketocarbonsäurederivate der allgemeinen Formel (III) eingesetzt werden, bei denen $R^1$ und $R^4$ gleich oder verschieden sein können und Alkylgruppe mit 1 bis 3 Kohlenstoffatomen bedeuten.

5. Verfahren nach einem oder mehreren der Ansprüche 1 bis 4, dadurch gekennzeichnet, daß als Erdalkalimetallverbindung Erdalkalimetalloxid eingesetzt wird.

6. Verfahren nach einem oder mehreren der Ansprüche 1 bis 5, dadurch gekennzeichnet, daß als Erdalkalimetallverbindung calciumoxid eingesetzt wird.

7. Verfahren nach einem oder mehreren der Ansprüche 1 bis 4, dadurch gekennzeichnet, daß als Erdalkalimetallverbindung Erdalkalihydroxid eingesetzt wird.

8. Verfahren nach einem oder mehreren der Ansprüche 1 bis 7, dadurch gekennzeichnet, daß die Umsetzung in Anwesenheit von Lösungsmittel durchgeführt wird.

9. Verfahren nach einem oder mehreren der Ansprüche 1 bis 8, dadurch gekennzeichnet, daß Acetessigsäurederivat mit der äquimolaren Menge an Erdalkalimetalloxid oder Erdalkalimetallhydroxid und Tetrahydrofuran und/oder Ethylenglycoldimethylether enthaltendem Lösungsmittel vermischt, auf die Siedetemperatur des Tetrahydrofuran und/oder Ethylenglycoldimethylether enthaltenden Lösungsmittels erhitzt und mit einer äquimolaren Menge, bezogen auf die Menge an eingesetztem Acetessigsäurederivat, eines Gemisches von Diketen enthaltend 4-Methylen-2-oxetan und 3-Methyl-4-methylen-2-oxetan versetzt wird.

**10.** Verwendung eines Gemisches enthaltend 6-Methyl-$\beta$-resorcylsäuremethylester und 3,6-Dimethyl-$\beta$-resorcylsäuremethylester als Riechstoff mit Eichenmooscharakter.

**Claims**

**1.** Process for the preparation of a $\beta$-resorcylic acid derivative of the general formula

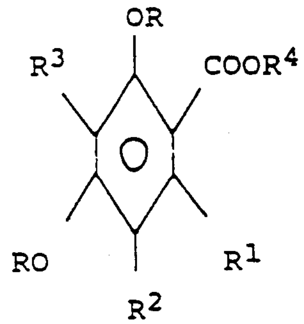

(I)

in which R, $R^2$ and $R^3$ can be identical or different and denote a hydrogen atom or an alkyl radical and $R^1$ and $R^4$ can be identical or different and denote an alkyl radical, by reacting a diketene of the general formula

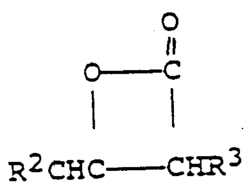

(II),

in which $R^2$ and $R^3$ have the abovementioned meaning, with a $\beta$-ketocarboxylic acid derivative of the general formula

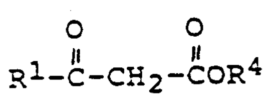

(III),

in which $R^1$ and $R^4$ have the abovementioned meaning, in the presence of an alkaline earth metal compound.

**2.** Process according to Claim 1, characterized in that a diketene of the general formula (II) in which $R^2$ and $R^3$ can be identical or different and denote a hydrogen atom or an alkyl group having 1 to 3 carbon atoms is employed.

**3.** Process according to either of Claims 1 or 2, characterized in that a diketene of the general formula (II) is prepared by reaction of a carboxylic acid halide of the general formula

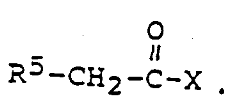

(IV)

in which X denotes a halogen atom and $R^5$ has one of the meanings of $R^2$ or $R^3$, in the presence of a

base.

4. Process according to any one of Claims 1 to 3, characterized in that a $\beta$-ketocarboxylic acid derivative of the general formula (III) in which $R^1$ and $R^4$ can be identical or different and denote an alkyl group having 1 to 3 carbon atoms is employed.

5. Process according to any one of Claims 1 to 4, characterized in that an alkaline earth metal oxide is employed as the alkaline earth metal compound.

6. Process according to any one of Claims 1 to 5, characterized in that calcium oxide is employed as the alkaline earth metal compound.

7. Process according to any one of Claims 1 to 4, characterized in that an alkaline earth metal hydroxide is employed as the alkaline earth metal compound.

8. Process according to any one of Claims 1 to 7, characterized in that the reaction is carried out in the presence of a solvent.

9. Process according to any one of Claims 1 to 8, characterized in that an acetoacetic acid derivative is mixed with the equimolar amount of an alkaline earth metal oxide or alkaline earth metal hydroxide and a solvent containing tetrahydrofuran and/or ethylene glycol dimethyl ether, the mixture is heated to the boiling point of the solvent containing tetrahydrofuran and/or ethylene glycol dimethyl ether, and an equimolar amount, based on the amount of acetoacetic acid derivative employed, of a mixture of diketene containing 4-methylene-2-oxetane and 3-methyl-4-methylene-2-oxetane is added.

10. Use of a mixture containing methyl 6-methyl-$\beta$-resorcylate and methyl 3,6-dimethyl-$\beta$-resorcylate as an odoriferous substance having an oak moss character.

**Revendications**

1. Procédé pour la préparation de dérivés d'acide $\beta$-résorcylique de formule générale

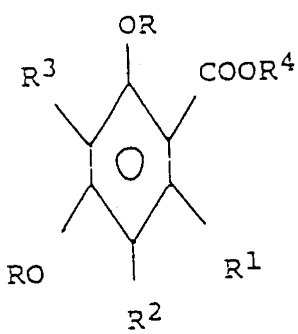

(I),

où R, $R^2$ et $R^3$ peuvent être identiques ou différents et représentent l'atome d'hydrogène ou un radical alkyle et $R^1$ et $R^4$ peuvent être identiques ou différents et représentent un radical alkyle,
par réaction de dicétène de formule générale

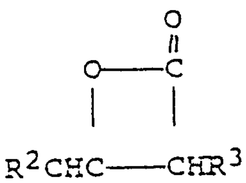

(II),

dans laquelle $R^2$ et $R^3$ ont la signification donnée ci-dessus, avec des dérivés d'acide $\beta$-cétocarboxylique de formule générale

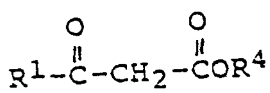

(III),

dans laquelle $R^1$ et $R^4$ ont la signification donnée ci-dessus, en Présence d'un composé de métal alcalino-terreux.

2. Procédé selon la revendication 1, caractérisé en ce qu'on utilise des dicétènes de formule générale (II) dans lesquels $R^2$ et $R^3$ peuvent être identiques ou différents et représenter un atome d'hydrogène ou un groupe alkyle avec 1 à 3 atomes de carbone.

3. Procédé selon la revendication 1 ou 2, caractérisé en ce qu'on prépare les dicétènes de formule générale (II) par réaction d'halogénures d'acides carboxyliques de formule générale

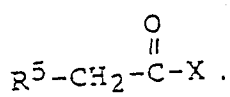

(IV),

où X représente l'atome d'halogène et $R^5$ a une des significations de $R^{2,}$ respectivement $R^3$, en présence d'une base.

4. Procédé selon une ou plusieurs des revendications 1 à 3, caractérisé en ce qu'on utilise des dérivés d'acide $\beta$-cétocarboxylique de formule générale (III) dans lesquels $R^1$ et $R^4$ peuvent être identiques ou différents et peuvent signifier un groupe alkyle avec 1 à 3 atomes de carbone.

5. Procédé selon une ou plusieurs des revendications 1 à 4, caractérisé en ce qu'on utilise en tant que composé de métal alcalino-terreux un oxyde de métal alcalino-terreux.

6. Procédé selon une ou plusieurs des revendications 1 à 5, caractérisé en ce qu'on utilise en tant que composé de métal alcalin l'oxyde de calcium.

7. Procédé selon une ou plusieurs des revendications 1 à 4, caractérisé en ce qu'on utilise en tant que composé de métal alcalino-terreux, un hydroxyde de métal alcalino-terreux.

8. Procédé selon une ou plusieurs des revendications 1 à 7, caractérisé en ce qu'on met en oeuvre la réaction en présence de solvants.

9. Procédé selon une ou plusieurs des revendications 1 à 8, caractérisé en ce qu'on mélange un dérivé d'acide acéto-acétique avec la quantité équimolaire d'oxyde de métal alcalino-terreux ou d'hydroxyde de métal alcalino-terreux et le solvant contenant le tétrahydrofuranne et/ou l'éther diméthylique de l'éthylèneglycol, on chauffe à la température d'ébullition du solvant contenant le tétrahydrofuranne et/ou l'éther diméthylique de l'éthylèneglycol et on ajoute une quantité équimolaire, par rapport à la quantité utilisée de dérivé d'acide acéto-acétique, d'un mélange de dicétène contenant le 4-méthylène-2-oxétane et le 3-méthyl-4-méthylène-2-oxétane.

10. Utilisation d'un mélange contenant l'ester méthylique de l'acide 6-méthyl-$\beta$-résorcylique et l'ester méthylique de l'acide 3,6-diméthyl-$\beta$-résorcylique en tant que substance odorante à caractère de mousse de chêne.